# EUROPÄISCHE PATENTSCHRIFT

(11) **EP 0 726 265 B1**
(45) Veröffentlichungstag und Bekanntmachung des Hinweises auf die Patenterteilung: **25.07.2001**
(21) Anmeldenummer: 96810062.8
(22) Anmeldetag: 30.01.1996
(51) Int. Cl.: C07D 313/14, A61K 31/335

(54) **Antineurodegenerativ wirksame 10-Aminoaliphatyl-dibenz- b,f oxepine**
10-Aminoaliphatyl-dibenz(b,f)oxepins with antineurodegenarative activity
10-Aminoaliphatyl-dibenz(b,f)oxépines avec une activité antineurodégénérative

(30) Priorität: 08.02.1995 CH 36795
(43) Veröffentlichungstag der Anmeldung: 14.08.1996
(73) Patentinhaber: Novartis AG, 4058 Basel (CH); Novartis-Erfindungen Verwaltungsgesellschaft m.b.H., 1235 Wien (AT)
(72) Erfinder: Betschart, Claudia, Dr., Takarazuka 665 (JP); Zimmermann, Kaspar, Dr., CH-4125 Riehen (CH)

(56) Entgegenhaltungen:
- DE-A- 1 793 521
- US-A- 3 100 207
- US-A- 3 928 383
- CHEMICAL ABSTRACTS, vol. 85, no. 11, 13.September 1976 Columbus, Ohio, US; abstract no. 77999v, Seite 533; XP002002842 & JP-A-75 160 274 (YAMANOUCHI)
- CHEMICAL ABSTRACTS, vol. 81, no. 19, 11.November 1974 Columbus, Ohio, US; abstract no. 120510k, Seite 526; XP002002843 & JP-A-07 451 289 (YAMANOUCHI)

## Beschreibung

Die Erfindung betrifft 10-Aminoaliphatyl-dibenz[b,f]oxepinen der Formel I 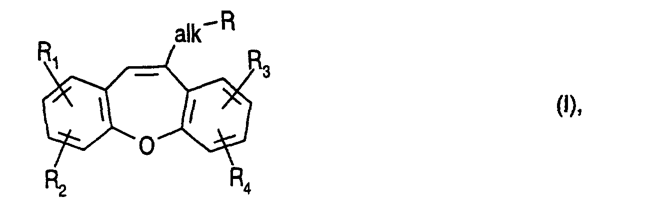 worin
alk Methylen bedeutet,
R Amino, unsubstituiertes oder durch C₁-C₄-Alkyl, C₁-C₄-Alkoxy, Halogen der Atomnummer bis und mit 35 und/oder Trifluormethyl substituiertes Phenyl-C₁-C₄-alkylamino, unsubstituiertes oder durch C₁-C₄-Alkyl, C₁-C₄-Alkoxy, Halogen der Atomnummer bis und mit 35 und/oder Trifluormethyl substituierte N-Phenyl-C₁-C₄-alkyl-N-C₁-C₄-alkyl-amino oder C₂-C₇-Alkenylamino, C₂-C₇-Alkinylamino, N-C₂-C₇-Alkenyl-N-C₁-C₄-Alkyl-amino, N-C₂-C₇-Alkinyl-N-C₁-C₄-alkyl-amino, darstellt und
R₁, R₂, R₃ und R₄ unabhängig voneinander für Wasserstoff, C₁-C₄-Alkyl, C₁-C₄-Alkoxy, Halogen der Atomnummer bis und mit 35 oder Trifluormethyl stehen,
und ihre Salze,
Verfahren zu ihrer Herstellung und diese enthaltende pharmazeutische Präparate.

Salze von Verbindungen der Formel I sind beispielsweise deren pharmazeutisch verwendbare Säureadditionssalze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Die Erfindung beruht auf der überraschenden Feststellung, dass Verbindungen der Formel I an neugeborenen Ratten in einer Versuchsanordnung nach Ausari et al., J. Neuroscience 13, 4042-4053 (1993) in Dosen von etwa 0.1 mg/kg s.c. und darunter eine ausgeprägte Schutzwirkung der Fazialismotoneuronen vor apoptotischem Zelltod sowie an ausgewachsenen Ratten in einer Versuchsanordnung nach Golowitz und Paterson, Soc. Neurosc. Abstr. 20, 246, 113.2 (1994) nach Verabreichung von 0.275 mg/kg. s.c. und darunter über 4 Tage eine ausgeprägte Schutzwirkung hippocampaler Pyramidalzellen vor Zelltod durch Verabreichung von Kainsäure aufweisen.

Die Verbindungen der Formel 1 und ihre pharmazeutisch verwendbaren Salze sind dementsprechend über ihre vorbekannte adrenolytische und zentraldämpfende Verwendbarkeit hinaus vorzüglich zur prophylaktischen oder therapeutischen Behandlung neurodegenerativer Erkrankungen, insbesondere solcher, bei denen apoptotischer Zelltod eine Rolle spielt, wie von cerebralen Ischämien, der Alzheimer'schen und der Parkinson'schen Erkrankung, von amyotropher Lateralsklerose, von Glaukoma sowie allgemeinen oder diabetischen peripheren Neuropathien vorzüglich geeignet.

Die Erfindung betrifft besonders Verbindungen der Formel I, worin
alk Methylen ist,
R C₂-C₇-Alkenylamino, wie Allylamino, Methallylamino oder But-2-enylamino, C₂-C₇-Alkinyl-amino, wie Propargylamino oder But-2-inylamino, N-C₂-C₇-Alkenyl-N-C₁-C₄-Alkyl-amino, wie N-Allyl-N-methyl-amino, N-Allyl-N-ethyl-amino, N-Methallyl-N-methyl-amino oder N-But-2-enyl-N-methyl-amino oder N-C₂-C₇-Alkinyl-N-C₁-C₄-alkyl-amino, wie N-Propargyl-N-methylamino, N-Propargyl-N-ethyl-amino oder N-But-2-inyl-N-methyl-amino darstellt,
R₁ und R₃ unabhängig voneinander Wasserstoff, C₁-C₄-Alkyl, wie Methyl, C₁-C₄-Alkoxy, wie Methoxy, Halogen der Atomnummer bis und mit 35, wie Chlor oder Brom, und/oder Trifluormethyl und
R₂, R₄ Wasserstoff bedeuten,
und ihre Salze, Verfahren zur Herstellung derselben und ihre Verwendung.

Die Erfindung betrifft in erster Linie Verbindungen der Formel I, worin
alk Methylen ist,
R C₂-C₇-Alkenylamino, wie Allylamino, Methallylamino oder But-2-enylamino, C₂-C₇-Alkinylamino, wie Propargylamino oder But-2-inylamino, N-C₂-C₇-Alkenyl-N-C₁-C₄-Alkyl-amino, wie N-Allyl-N-methyl-amino, N-Allyl-N-ethyl-amino, N-Methallyl-N-methyl-amino oder N-But-2-enyl-N-methyl-amino, N-C₂-C₇-Alkinyl-N-C₁-C₄-alkyl-amino, wie N-Propargyl-N-methylamino, N-Propargyl-N-ethyl-amino oder N-But-2-inyl-N-methyl-amino, oder unsubstituiertes oder durch C₁-C₄-Alkyl, wie Methyl, C₁-C₄-Alkoxy, wie Methoxy, Halogen der Atomnummer bis und mit 35, wie Chlor oder Brom, und/oder Trifluormethyl substituiertes Phenyl-C₁-C₄-alkylamino, wie Benzylamino oder Phenethylamino, darstellt und
R₁, R₂, R₃ und R₄ Wasserstoff bedeuten,
und ihre Salze, Verfahren zu ihrer Herstellung und ihre Verwendung.

Die Erfindung betrifft namentlich
N-(Dibenz[b,f]oxepin-10-ylmethyl)-N-methyl-N-prop-2-inyl-amin;
N-Allyl-N-(dibenz[b,f]oxepin-10-ylmethyl)-amin;
N-Benzyl-N-(dibenz[b,f]oxepin-10-ylmethyl)-amin;
N-(Dibenz[b,f]oxepin-10-ylmethyl)-N-prop-2-inyl-amin;
N-Allyl-N-(dibenz[b,f]oxepin-10-ylmethyl)-N-methyl-amin;
N-(Dibenz[b,f]oxepin-10-ylmethyl)amin;
N-(1-Chlor-dibenz[b,f]oxepin-10-ylmethyl)-N-methyl-N-prop-2-inyl-amin;
N-Methyl-N-prop-2-inyl-N-(3-trifluormethyl-dibenz[b,f]oxepin-10-ylmethyl)-amin;
N-(7-Chlor-dibenz[b,f]oxepin-10-ylmethyl)-N-methyl-N-prop-2-inyl-amin;
N-(8-Methoxy-dibenz[b,f]oxepin-10-ylmethyl)-N-methyl-N-prop-2-inyl-amin;
N-(8-Tertiärbutyl-dibenz[b,f]oxepin-10-ylmethyl)-N-methyl-N-prop-2-inyl-amin;
N-(6-Chlor-dibenz[b,f]oxepin-10-ylmethyl)-N-methyl-N-prop-2-inyl-amin;
N-(1-Fluor-dibenz[b,f]oxepin-10-ylmethyl)-N-methyl-N-prop-2-inyl-amin
N-Benzyl-N-(dibenz[b,f]oxepin-10-ylmethyl)-N-methyl-amin;
N-(Dibenz[b,f]oxepin-10-ylmethyl)-N-propyl-N-benzyl-amin; und
N-(7-Chlor-dibenz[b,f]oxepin-10-ylmethyl)-N-methyl-N-prop-2-inyl-amin.

Das Verfahren zur Herstellung der Verbindungen der Formel I und ihrer Salze ist dadurch gekennzeichnet, dass man eine Verbindung der Formel II 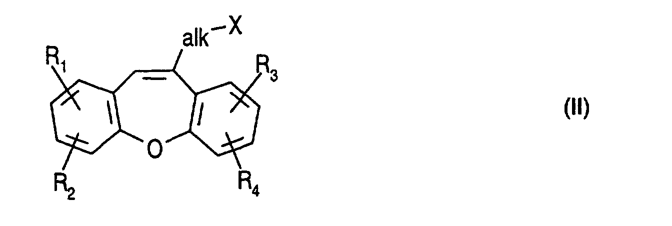 mit einer Verbindung der Formel III

Y-R (III)

kondensiert, worin einer der Reste X und Y reaktionsfähiges verestertes Hydroxy und der andere gegebenenfalls intermediär geschütztes Amino bedeutet und R, R₁, R₂, R₃ und R₄ die angegebenen Bedeutungen haben, und die gegebenenfalls intermediär eingeführte Aminoschutzgruppe wieder abspaltet, und gewünschtenfalls jeweils eine verfahrensgemäss erhältliche Verbindung in eine andere Verbindung der Formel I umwandelt, ein verfahrensgemäss erhältliches Isomerengemische in die Komponenten auftrennt und das gewünschte Isomere isoliert und/oder ein verfahrensgemäss erhältliches Salz in die freie Verbindung oder eine verfahrensgemäss erhältliche freie Verbindung in ein Salz überführt.

Reaktionsfähiges veresterte Hydroxy in Ausgangsstoffen der Formel II bzw. III ist beispielsweise mit einer Halogenwasserstoffsäure oder einer organischen Sulfonsäure verestertes Hydroxy, wie Halogen, z.B. Chlor, Brom oder Jod, gegebenenfalls durch Niederalkyl, Halogen und/oder Nitro substituiertes Benzolsulfonyloxy, wie Benzolsulfonyloxy, p-Brombenzolsulfonyloxy oder p-Toluolsulfonyloxy, oder Niederalkansulfonyloxy, wie Methansulfonyloxy.

Die Umsetzung von Verbindungen der Formeln II und III erfolgt in üblicher Weise, beispielsweise in Gegenwart eines basischen Kondensationsmittels, wie einer tertiären oder sterisch gehinderten binären organischen Stickstoffbase, wie eines Triniederalkylamins oder sterisch gehinderten Diniederalkylamins, wie Triethylamin oder Diisopropylamin, oder einer heteroaromatischen Base, wie Pyridin oder Dimethylaminopyridin, vorteilhaft in einem organischen Lösungsmittel, wie Toluol, und erforderlichenfalls unter Kühlen oder Erwärmen, z.B. im Temperaturbereich von etwa 0° bis etwa 80° C.

Als Aminoschutzgruppen kommen für den intermediären Schutz primärer Aminogruppen üblichen Aminoschutzgruppen in Betracht, insbesondere solvolytisch abspaltbare Aminoschutzgruppen, in Betracht. Solche sind beispielsweise von einer Carbonsäure oder einem Halbester der Kohlensäure abgeleitete Acylgruppen, wie gegebenenfalls halogeniertes Niederalkanoyl, beispielsweise Niederalkanoyl, wie Formyl, Acetyl oder Pivaloyl, Polyhalogenniederalkanoyl, wie Trifluoracetyl, Niederalkoxycarbonyl, wie Methoxycarbonyl, Ethoxycarbonyl, Isopropyloxycarbonyl, oder Tertiärbutyloxycarbonyl, oder gegebenenfalls substituiertes Phenylniederalkoxycarbonyl, wie Benzyloxycarbonyl, ferner Silylgruppen, wie Triniederalkylsilyl, z.B. Trimethylsilyl.

Die Abspaltung dieser Aminoschutzgruppen erfolgt in üblicher Weise, beispielsweise durch Behandlung mit einem Solvolysemittel, wie mit Wasser in Gegenwart einer Säure, z.B. einer wässrigen Mineralsäure, wie Halogenwasserstoffsäure, oder einem Alkalimetallhydroxid, wie Natronlauge oder Kalilauge, insbesondere für die Abspaltung einer Sulfonsäure, wie Methansulfonsäure in einem halogenierten Kohlenwassertstoff, wie Dichlormethan, oder, insbesondere für die Abspaltung von Formyl, einer geeigneten Silylverbindung, wie einem Triniederalkylsilylhalogenid, wie Trimethylsilylbromid, oder einem Disilazan, wie Hexamethyldisilazan.

Die Ausgangsstoffe der Formeln II und III sind bekannt oder können in Analogie zu der Bildungsweise bekannter Verbindungen der Formeln II und III hergestellt werden.

So erhält man Verbindungen der Formel II, worin X reaktionsfähig verestertes Hydroxy bedeutet, beispielsweise indem man Verbindungen der Formeln V und VI 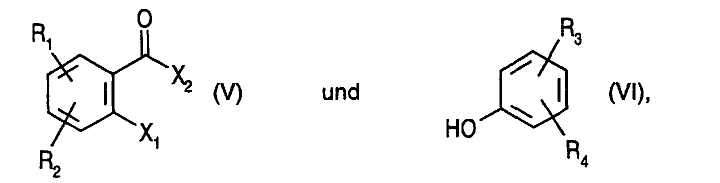 worin X₁ Halogen und X₂ Wasserstoff oder Hydroxy bedeutet, in üblicher Weise miteinander kondensiert, wobei man wobei man vorzugsweise im Temperaturbereich von etwa 100° C bis etwa 180° C arbeitet und, ausgehend von Verbindungen der Formel V, worin X₂ Hydroxy und X₁ beispielsweise Chlor bedeutet, vorzugsweise in Gegenwart von Kupfer/Kupfer-1-jodid und ausgehend von Verbindungen der Formel V, worin X₂ Wasserstoff und X₁ beispielsweise Fluor bedeutet, vorzugsweise in Gegenwart von Kaliumcarbonat in Dimethylacetamid arbeitet, in der erhaltenen Verbindungen der Formel VII 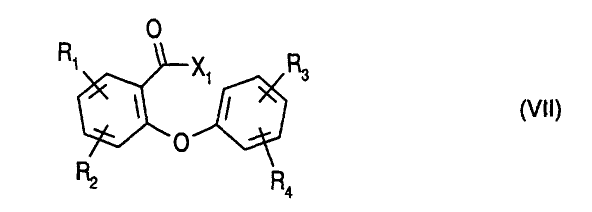 die Gruppe -C(=P)-X₁ in üblicher Weise, beispielsweise durch Behandeln mit einem Dileichtmetallhydrid, wie Lithiumaluminiumhydrid in Tetrahydrofuran, zu Hydroxymethyl reduziert, die Hydroxymethylgruppe in üblicher Weise, beispielsweise durch Erwärmen mit einer Halogenwasserstoffsäure, insbesondere Bromwasserstoffsäure, in Halogenmethyl überführt, das Halogenatom in üblicher Weise, beispielsweise durch Behandeln mit einem Alkalimetallcyanid, wie Natriumcyanid in Ethanol, durch Cyano ersetzt und die erhaltene Verbindung der Formel VIII 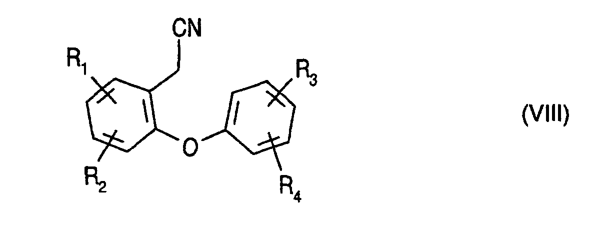 in üblicher Weise, beispielsweise in Gegenwart eines Alkallimetallalkanolates, wie Natriummethanolat, mit einem Oxalsäurediniederalkylester, z.B. Diethyloxalat, umsetzt, sauer aufarbeitet, in der erhaltenen Verbindung der Formel IX 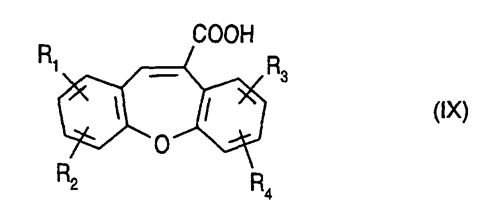 die Carboxygruppe in üblicher Weise, beispielsweise durch Behandeln mit einem Halogenameisensäureester, wie Isobutylchlorformat, in Gegenwart einer Stickstoffbase, wie N-Methylmorpholin, vorzugsweise in einem etherartigen Lösungsmittel, wie Dimethoxymethan, und anschliessend mit einem Dileichtmetallhydrid, wie Natriumborhydrid in Wasser, zu Hydroxymethyl reduziert und die erhaltene Verbindung der Formel X 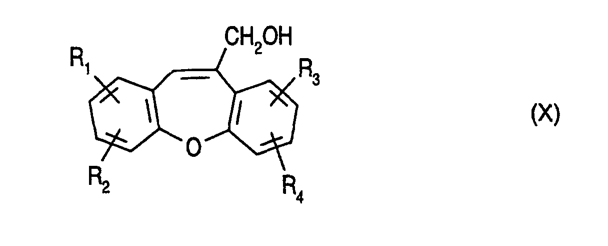 mit einem die Gruppe X einführendem Mittel, wie einer Halogenwasserstoffsäure oder einem Sulfonylhalogenid, wie Methansulfonylchlorid, behandelt.

Verbindungen der Formel II, worin X gegebenenfalls geschütztes Amino bedeutet, können beispielsweise aus den wie vorstehend erhaltenen reaktionsfähigen Estern der Formel II, worin X reaktionsfähiges verestertes Hydroxy bedeutet, durch Umsetzung mit Ammoniak in übliche Weise, beispielsweise durch Behandlung mit einer gesättigten Lösung von Ammoniak in Methanol, und gewünschtenfalls anschliessende Einführung der Aminoschutzgruppe in üblicher Weise erhalten werden.

Verfahrensgemäss erhältliche Verbindungen können in üblicher Weise in andere Verbindungen der Formel I überführt werden.

So kann man in Verbindungen der Formel I, worin R unsubstituiertes Amino bedeutet, die Aminogruppe in üblicher Weise durch einen oder zwei gleiche oder verschiedene Reste substituieren. In analoger Weise kann man auch in Verbindungen der Formel I, worin R monosubstituiertes Amino bedeutet, einen weiteren Rest einführen.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und emeute Versalzung.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.
Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel 1 enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Bomeol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die Erfindung betrifft als weiteren bevorzugten Erfindungsgegenstand pharmazeutische Präparate, die die erfindungsgemässen Verbindungen der Formel I oder pharmazeutisch verwendbare Salze derselben als Wirkstoffe enthalten.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter, wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, femer Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Laktose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Traganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glyzerin oder Sorbit. Die Steckkapseln können den Wirkstoff in Form eines Granulates, z.B. im Gemisch mit Füllstoffen, wie Laktose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykole oder höhere Alkanole. Femer können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung durch Infusion und/oder Injektion eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Suspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Suspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 500 mg zu veranschlagen.

Die nachfolgen Beispiele dienen zur Illustration der Erfindung; Temperaturen sind in Celsiusgraden, Drucke in mbar angegeben.

### Beispiel 1: N-(Dibenz[b,f]oxepin-10-ylmethyl-N-methyl-N-prop-2-inyl-amin

Methyl-propargylamin (4.5 g, 65 mMol) wird in Benzol (75 ml) und Methanol (25 ml) gelöst. Bei 40° C tropft man innerhalb einer halben Stunde die Lösung von 10-Brommethyldibenz[b,f]oxepin (7.0 g, 25 mMol) in Benzol (25 ml) zu. Nach vollendeter Zugabe lässt man nochmals eine halbe Stunde bei 40-50°C rühren. Man giesst auf Wasser, wäscht dreimal mit Wasser und extrahiert dann mit 5%iger Methansulfonsäure. Die saure wässrige Phase wird mit konzentriertem Ammoniak basisch gestellt und mit Diethylether extrahiert. Die etherische Phase wird über Natriumsulfat getrocknet und eingedampft. Kristallisation des Rückstands aus Petrolether gibt N-(Dibenz[b,f]oxepin-10-ylmethyl-N-methyl-N-prop-2-inylamin *alias* 10-(N-Propargyl-N-methyl-amino)methyldibenz[b,f]oxepin (5.3 g, 77%). Schmelzpunkt: 66-67°C.

### Beispiel 2: N-Allyl-N-(dibenz[b,f]oxepin-10-ylmethyl)-amin-hydrochlorid

Zu einer Lösung von 0.3 ml (4 mMol) Allylamin in 0.9 ml wasserfreiem Methanol wird bei 50°C eine Lösung von 0.5 ml (1.74 mMol) 10-Bromomethyl-dibenz[b,f]oxepin zugespritzt und während 30 Minuten gerührt. Man versetzt mit Tertiärbutyl-methyl-ether (Tertiärbutyl-methyl-ether) und Essigsäureethylester, extrahiert 3x mit je 20 ml 1N Salzsäure, stellt die vereinigte wässrige Phase mit Kaliumhydroxid-Plätzchen basisch, extrahiert 2x mit Essigsäureethylester, trocknet die org. Phasen über Natriumsulfat und engt ein. Das rohe Amin (180 mg) wird in 2 ml Diethylether mit 0.35 ml (0.7 mMol) 2N-etherischer Salzsäure versetzt, das ausfallende, weisse Hydrochlorid mit Diethylether gewaschen und am HV bei 40°C getrocknet. Man erhält 200 mg (667 µMol) = 39% der Titelverbindung *alias* 10-Allylaminomethyldibenz[b,f]oxepin als weisse Kristalle; Schmelzpunkt: 148-158°C; ¹H-NMR (CD₃OD, 200 MHz): 3.74 (d, 2H); 4.35 (d, 2H); 5.55 (m, 2H); 5.95 (m, 1H); 7.20-7.58 (m, 9H); MS: 263 (M⁺, freie Base), 222, 208, 181, 165, 152.

### Beispiel 3: N-Allyl-N-(dibenz[b,f]oxepin-10-ylmethyl)-N-methyl-amin-hydrochlorid

Herstellung analog Beispiel 2 aus 10-Bromomethyl-dibenz[b,f]oxepin und N-Methyl-allylamin. Ausbeute: 71%; Schmelzpunkt: 153-156°C; ¹H-NMR (CD₃OD, 200 MHz): 2.85 (s, 3H); 3.86 (d, 2H); 4.50 (sbr, 2H); 5.60 (d, 1H); 5.68 (s, 1H); 6.00 (m, 1H); 7.20-7.60 (m, 9H); MS: 277 (M⁺, freie Base), 208, 181, 152.

### Beispiel 4: N-(Dibenz[b,f]oxepin-10-ylmethyl-N-methyl-N-prop-2-inyl-amin

Herstellung analog Beispiel 2 aus 10-Bromomethyl-dibenz[b,f]oxepin und N-Methyl-N-propargyl-amin, als freie Base, chromatographiert an Kieselgel mit Hexan/Essigsäureethylester = 4:1 und kristallisiert aus wenig Petrolether. Ausbeute: 74%; Schmelzpunkt: 67-68°C; ¹H-NMR (CDCl₃, 300 MHz): 2.30 (d, 1H); 2.42 (s, 3H); 3.48 (t, 2H); 3.65 (s, 2H); 6.90 (d, 1H); 7.08-7.36 (m, 7H); 7.56 (m, 1H); MS: 275 (M⁺), 232, 208, 181, 165, 152; Analyse: C 82.77% (82.88); H 6.18% (6.22); N 4.99% (5.09).
Die Titelverbindung ist identisch mit dem Produkt gemäss Beispiel 1.

### Beispiel 5: N-(Dibenz[b,f]oxepin-10-ylmethyl-N-methyl-N-prop-2-inyl-amin-oxalat

Herstellung analog Beispiel 2; Oxalatsalz aus freier Base mit Oxalsäure in Ethanol. Schmelzpunkt: 202-205°C; Analyse: C 68.79% (69.03); H 5.29% (5.24); N 3.86% (3.83).

### Beispiel 6: N-(Dibenz[b,f]oxepin-10-ylmethyl)amin (als Hydrochlorid)

Zu 30 ml NH₃-gesättigtem Methanol tropft man 1.0 g (3.48 mMol) 10-Bromomethyldibenz[b,f]oxepin in 3 ml Toluol bei 40°C, lässt bei 35-50°C während 1 Stunde und bei Raumtemperatur über Nacht rühren. Man zieht das Lösungsmittel teilweise ab, nimmt das Reaktionsgemisch in Tertiärbutyl-methyl-ether auf, wäscht mit 0.1 N Natronlauge. und extrahiert mit 1N Salzsäure. Man stellt die wässrige Phase mit Natriumhydroxid-Plätzchen basisch, extrahiert mit Tertiärbutyl-methyl-ether, trocknet die org. Phase über Natriumsulfat und zieht das Lösungsmittel ab. Man erhält 349 mg (1.56 mMol) = 45% N-(Dibenz[b,f]oxepin-10-ylmethyl)amin als hellgelbes Öl; DC (KG; Essigsäureethylester; UV): R_{f} = 0.09.

### Beispiel 7: N-(Dibenz[b,f]oxepin-10-ylmethyl)-N-prop-2-inyl-amin -hydrochlorid

Man rührt ein Gemisch von 1.1 g (3.044 mMol) N-(Dibenz[b,f]oxepin-10-ylmethyl-prop-2-inyl-carbaminsäure-tertiärbutylester und 1.45 ml Methansulfonsäure in 1 ml Dioxan und 9 ml Dichlormethan während 1 Stunde bei Raumtemperatur, versetzt mit 2N Natronlauge. und extrahiert 2x mit Dichlormethan. Man engt die org. Phase am Rotationsverdampfer ein, nimmt in Essigsäureethylester auf, extrahiert 3x mit 1N Salzsäure, stellt die wässrige Phase mit Kaliumhydroxid-Plätzchen basisch, extrahiert 3x mit Dichlormethan, trocknet über Natriumsulfat und engt ein. Das rohe hellbraune Öl wird in 2N etherischer Salzsäure aufgelöst und eingeengt. Das rohe Hydrochlorid (beige Kristalle) wird aus Essigsäureethylester/Methanol umkristallisiert. Man erhält 386 mg (1.30 mMol) = 42% N-(Dibenz[b,f]oxepin-10-ylmethyl)-N-prop-2-inyl-amin-hydrochlorid *alias* 10-Propargylaminomethyldibenz[b,f]oxepin-hydrochlorid als weisse Kristalle; Schmelzpunkt: 181-183°C; MS: 261 (M⁺, freie Base), 222, 181, 165, 152.

Das Ausgangsmaterial kann beispielsweise auf folgende Weise hergestellt werden:

### a) Dibenz[b,f]oxepin-10-ylmethyl-carbaminsäure-tertiärbutylester

Zu einer Lösung von 2.3 g (10.3 mMol) N-(Dibenz[b,f]oxepin-10-ylmethyl)amin in 20 ml Dichlormethan wird 2.25 g (10.3 mMol) Di-tertiärbutyl-dicarbonat (BOC)₂O bei Raumtemperatur zugefügt, 30 Minuten gerührt und am Rotationsverdampfer vom Lösungsmittel befreit. Man erhält 3.61 g Dibenz[b,f]oxepin-10-ylmethyl-carbaminsäuretertiärbutylester als rohes, gelbes Öl, das beim Stehenlassen fest wird.
DC (KG; Essigsäureethylester/Hexan = 9:1; UV): R_{f} = 0.36.

### b) Dibenz[b,f]oxepin-10-ylmethyl-prop-2-inyl-carbaminsäure-tertiärbutylester

Man legt 1.0 g (3.091 mMol) Dibenz[b,f]oxepin-10-ylmethyl-carbaminsäure-tertiärbutylester in 10 ml Dimethylformamid vor, gibt bei Raumtemperatur 0.22 g (4.636 mMol) 55%-ige

Natriumhydrid-Suspension (in Öl) zu, rührt 15 Minuten und tropft dann 0.279 ml (3.709 mMol) Propargylbromid bei Raumtemperatur zu. Nach 1 Stunde gibt man vorsichtig Wasser und wenig Sole zu und versetzt mit Tertiärbutyl-methyl-ether. Die organische Phase wird 4x mit Wasser gewaschen, über Natriumsulfat getrocknet und eingeengt. Man erhält 1.106 g (3.06 mMol) = 98.8% Dibenz[b,f]oxepin-10-ylmethyl-prop-2-inyl-carbaminsäuretertiärbutylester als braunes Öl; DC (KG; Essigsäureethylester/Hexan = 9:1; UV): R_{f} = 0.45.

### Beispiel 8: N-(1-Chlor-dibenz[b,f]oxepin-10-ylmethyl)-N-methyl-N-prop-2-inyl-aminhydrochlorid

Herstellung analog Beispiel 2 aus 10-Brommethyl-1-chlor-dibenz[b,f]oxepin mit N-Methyl-N-propargyl-amin. Ausbeute: 75%; Schmelzpunkt: nicht bestimmt, (weisser Schaum) ;
¹H-NMR (CDCl₃, 200 MHz): 2.35 (t, 1H); 2.45 (s, 3H); 3.51 (d, 2H); 3.70 (s, 2H); 7.10-7.40 (m, 7H); 7.61 (m, 1H).

Das Ausgangsmaterial kann beispielsweise auf folgende Weise hergestellt werden:

### a) 2-Chlor-6-phenoxy-benzaldehyd

Eine Mischung von 15.8 g (100 mMol) 2-Chlor-6-Fluorbenzaldehyd, 9.4 g (100 mMol) Phenol und 20.7 g (150 mMol) Kaliumcarbonat in 150 ml Dimethylacetamid wird während 4 h unter Rückfluss erhitzt. Man lässt abkühlen, versetzt mit Wasser und extrahiert 3x mit Tertiärbutyl-methylether. Die org. Phasen werden mit 2N Natronlauge und Sole gewaschen, über Natriumsulfat getrocknet und eingeengt. Kugelrohrdestillation (175°C, 0.04 mbar) liefert 19.46 g (83.64 mMol) = 83.6% 2-Chlor-6-phenoxy-benzaldehyd als gelbes Öl; ¹H-NMR (CDCl₃, 200 MHz): 6.75-7.45 (m, 8H); 10.58 (s, 1H).

### b) (2-Chlor-6-phenoxy-phenyl)methanol

Zu einer Suspension von 4.42 g (116.5 mMol) Lithiumaluminiumhydrid in ca. 150 ml Tetrahydrofuran wird 19.0 g (77.67 mMol) 2-Chlor-6-phenoxy-benzylaldehyd in ca. 40 ml Tetrahydrofuran bei Raumtemperatur innert 30 Minuten zugetropft. Danach wird für 4 h unter Rückfluss erhitzt, abgekühlt und mit 4.4 ml Wasser, 4.4 ml 4N Natriumhydroxid und 13.2 ml Wasser hydrolysiert. Das Reaktionsgemisch wird während 30 Minuten unter Rückfluss gekocht, gekühlt, abfiltriert, das Nutschgut 3x in Essigsäureethylester aufgenommen, 15 Minuten unter Rückfluss gekocht und filtriert. Die vereinigten Filtrate werden eingeengt. Man erhält 17.97 g (76.57 mMol) = 94% rohes (2-Chlor-6-phenoxyphenyl)-methanol als gelbes Öl;
DC (KG; Essigsäureethylester/Hexan = 1:1; UV): R_{f} = 0.68; ¹H-NMR (CDCl₃, 200 MHz): 4.90 (s, 2H); 6.76-7.40 (m, 8H).

### c) (2-Chlor-6-phenoxy-phenyl)-brom-methan

17.5 g (70.94 mMol) (2-Chlor-6-phenoxy-phenyl)-methanol wird in 150 ml 48%-iger Bromwasserstoffsäure während 3 h unter Rückfluss erhitzt. Man kühlt ab, versetzt mit Wasser und extrahiert 3x mit Essigsäureethylester. Die org. Phasen werden mit Sole gewaschen, über Natriumsulfat getrocknet und eingeengt. Man erhält 21.21 g (>100%) rohes (2-Chlor-6-phenoxy-phenyl)-brom-methan als gelbes Öl; DC (KG; Essigsäureethylester/Hexan =1:1; UV): R_{f} = 0.70; ¹H-NMR (CDCl₃, 200 MHz): 4.78 (s, 2H); 6.70-7.41 (m, 8H).

### d) (2-Chlor-6-phenoxy-phenyl)acetonitril

Zu einer Lösung von 4.5 g (91.74 mMol) Natriumcyanid in 9.2 ml Wasser und 2.3 ml Ethanol wird bei 80°C eine Lösung von 21.0 g (70.57 mMol) (2-Chlor-6-phenoxy-phenyl)-brom-methan in 16.5 ml Ethanol zugetropft und das Reaktionsgemisch während 4 Stunden unter Rückfluss gekocht. Nach dem Abkühlen wird am Rotationsverdampfer eingeengt, der Rückstand in Essigsäureethylester aufgenommen und die org. Phase 2x mit Wasser und 1x mit Sole gewaschen, über Natriumsulfat getrocknet und eingeengt. Man chromatographiert an Kieselgel mit Essigsäureethylester und erhält 10.82 g (44.40 mMol) = 63% (2-Chlor-6-phenoxy-phenyl)-acetonitril als braunes Öl; DC (KG; Essigsäureethylester; UV): R_{f} = 0.42; ¹H-NMR (CDCI3, 200 MHz): 3.96 (s, 2H); 6.74-7.46 (m, 8H).

### e) 1-Chlor-dibenz[b,f]oxepin-10-carbonsäure

Zu einer frisch zubereiteten Natriumethanolatlösung (1.5 g (53.72 mMol) Natrium in 50 ml Ethanol) wird bei Raumtemperatur 10.82 g (44.40 mMol) (2-Chlor-6-phenoxy-phenyl)-acetonitril und 7.85 g (53.72 mMol) Oxalsäurediethyl-ester zugegeben und während 18 Stunden gerührt. Man stellt mit 1n Salzsäure sauer, engt am Rotationsverdampfer ein, extrahiert 2x mit Essigsäureethylester und wäscht die org. Phase mit Sole. Man trocknet über Natriumsulfat getrocknet, engt ein und erhält 15.99 g rohes Zwischenprodukt (3-(2-Chlor-6-phenoxy-phenyl)-2-hydroxy-4-nitrilo-but-2-en-säure-ethyl-ester). 15.0 g (ca. 43.6 mMol) des obigen Zwischenproduktes werden in 105 ml Eisessig vorgelegt, 20 Minuten gerührt, mit 51.9 ml Wasser und 51.9 ml Schwefelsäure langsam versetzt, während 4 Stunden auf Rückfluss erhitzt und danach 18 Stunden bei Raumtemperatur gerührt. Man extrahiert das Reaktionsgemisch mit Essigsäureethylester, wäscht die org. Phase mit Wasser, 3x mit 4N Natriumhydroxid und extrahiert die wässrige Phase 1x mit Essigsäureethylester. Die wässrigen Phasen werden mit konzentrierter Salzsäure sauer gestellt, 3x mit Essigsäureethylester extrahiert, die org. Phase über Natriumsulfat getrocknet und eingeengt. Man erhält 7.71 g (28.27 mMol) = 60.3% 1-Chlordibenz[b,f]oxepin-10-carbonsäure als gelben Feststoff; DC (KG; Essigsäureethylester/Hexan = 1:1; UV): R_{f} = 0.21; ¹H-NMR (CDCl₃, 200 MHz): 7.15-7.47 (m, 7H); 7.68 (m, 1H); 8.39 (s, 1H).

### f) (1-Chlor-dibenz[b,f]oxepin-10-yl)methanol

Zu einer Lösung von 5.0 g (18.33 mMol) 1-Chlor-dibenz[b,f]oxepin-10-carbonsäure in Dimethoxyethan wird bei -15°C 2.0 ml (18.33 mMol) N-Methylmorpholin und 2.4 ml (18.33 mMol) Isobutylchlorformat zugetropft. Nach 5 Minuten wird filtriert und zum Filtrat bei -15°C eine Lösung von 1.39 g (36.67 mMol) Natriumborhydrid in 15 ml Wasser zugetropft. Man rührt 15 Minuten bei -15°C, gibt dann 35 ml 1N Salzsäure zu und lässt auf Raumtemperatur erwärmen. Das Reaktionsgemisch wird mit Natriumhydroxid basisch gestellt und 4x mit Essigsäureethylester extrahiert. Die org. Phase wird 1x mit Wasser gewaschen, über Natriumsulfat getrocknet und eingeengt. Man erhält 4.58 g (17.7 mMol) = 96.6% rohes (1-Chlor-dibenz[b,f]oxepin-10-yl)-methanol als gelbes Öl; DC (KG; Essigsäureethylester/Hexan = 1:1; UV): R_{f} = 0.46; ¹H-NMR (CDCl₃, 200 MHz): 2.42 (sbr, 1H); 4.71 (s, 2H); 7.10-7.45 (m, 7H).

### g) 10-Brommethyl-1-chlor-dibenz[b,f]oxepin

4.58 g (17.70 mMol) (1-Chlor-dibenz[b,f]oxepin-10-yl)-methanol wird in 50 ml 48%-iger Bromwasser-stoffsäure während 2 Stunden unter Rückfluss erhitzt. Man kühlt ab, versetzt mit Wasser und extrahiert 3x mit Essigsäureethylester. Die org. Phasen werden mit Sole gewaschen, über Natriumsulfat getrocknet und eingeengt. Man erhält 5.57 g Rohprodukt, welches beim Stehenlassen (3 Tage) fest wird. Durch Umkristallisation aus Tertiärbutylmethylether/Hexan erhält man 2.205 g (6.86 mMol) = 38.7% 10-Brommethyl-1-chlordibenz[b,f]oxepin als hellbeige Kristalle; DC (KG; Essigsäureethylester; UV): R_{f} = 0.73; ¹H-NMR (CDCI3, 200 MHz): 4.60 (s, 2H); 7.15-7.58 (m, 8H).

### Beispiel 9: N-Methyl-N-prop-2-inyl-N-(3-trifluormethyl-dibenz[b,f]oxepin-10-ylmethyl)-amin

Herstellung analog Beispiel 2 aus 10-Bromomethyl-3-trifluormethyl-dibenz[b,f]oxepin und N-Methyl-propargylamin. Als freie Base chromatographiert an Kieselgel mit Hexan/Essigsäureethylester = 1:1 und kristallisiert aus wenig Petrolether;; Ausbeute: 56%; Schmelzpunkt: 66-68°C;
¹H-NMR (CDCl₃, 200MHz): 1.80 (t, 1H); 2.41 (s, 3H); 3.46 (d, 2H); 3.65 (s, 2H); 6.91 (sbr, 1H); 7.15-7.58 (m, 7H) ; MS: 343 (M⁺), 342, 300, 276, 249, 205,178, 152.

Das Ausgangsmaterial kann beispielsweise auf folgende Weise hergestellt werden:

### a) 2-Phenoxy-4-trifluormethyl-benzaldehyd

Herstellung analog Beispiel 8 aus 2-Fluor-4-trifluormethyl-benzaldehyd und Phenol. Ausbeute: 78%, helle Kristalle, kristallisiert aus Hexan; Schmelzpunkt: 57-59°C; DC (KG; EssigsÄureethylester/Hexan = 1:1; UV): R_{f} = 0.71; ¹H-NMR (CDCl₃, 200MHz): 7.08-7.50 (m, 7H); 8.05 (d, 1H); 10.60 (s, 1H).
MS: 266/265 (M⁺), 217, 188.

### b) 3-Trifluormethyl-dibenz[b,f]oxepin-10-carbonsäure

Ein Gemisch von 10.0 g (37.59 mMol) 2-Phenoxy-4-trifluormethyl-benzaldehyd, 10.09 g (56.40 mMol) Hippursäure und 3.70 g (45.10 mMol) Natriumacetat im 38 ml Acetanhydrid wird während 80 min auf 85°C erwärmt, dann abgekühlt auf 32°C, mit 19 ml Wasser versetzt und während 30 Minuten auf 65°C erwärmt. Nach Kühlung auf ca. 5°C tropft man 19 ml konzentrierter Schwefelsäure zu und erhitzt danach für 2 Stunden unter Rückfluss (Bad: 140°C). Die beim Abkühlen entstandene, braune Fällung wird filtriert, mit 50%-iger Essigsäure gewaschen. Mit Wasser Neutralwaschen und Trocknen ergibt 7.42 g (24.23 mMol) = 65% 3-Trifluormethyl-dibenz[b,f]oxepin-10-carbonsäure als beige Kristalle; Schmelzpunkt: 180°C; ¹H-NMR (CDCl₃, 200MHz): 7.2-7.65 (m, 8H); 8.09 (s, 1H).

### c) (3-Trifluormethyl-dibenz[b,f]oxepin-10-yl)-methanol

Herstellung analog Beispiel 8f) aus 3-Trifluormethyl-dibenz[b,f]oxepin-10-carbonsäure. Produkt kristallisiert aus Tertiärbutyl-methylether/Hexan; Ausbeute: 75.2%; weisse Kristalle; DC (KG; Essigsäureethylester/Hexan = 1:1; UV): R_{f} = 0.31; ¹H-NMR (CDCl₃, 200MHz): 2.76 (sbr, 1H); 4.72 (s, 2H); 6.96 (s, 1H); 7.16-7.48 (m, 7H).

### d) 10-Brommethyl-3-trifluormethyl-dibenz[b,f]oxepin

Herstellung analog Beispiel 8g) aus (3-Trifluormethyl-dibenz[b,f]oxepin-10-yl)-methanol. Produkt kristallisiert aus Hexan; Ausbeute: 92% weisse Kristalle; DC (KG; Essigsäureethylester/Hexan = 1:1; UV): R_{f} = 0.70; ¹H-NMR (CDCl₃, 200MHz): 4.55 (s, 2H); 7.04 (s, 1H); 7.20-7.60 (m, 7H) ; MS: 356/354 (M⁺), 275, 249, 219, 206, 205, 178, 176.

### Beispiel 10: N-(7-Chlor-dibenz[b,f]oxepin-10-ylmethyl)-N-methyl-N-prop-2-inyl-aminhydrochlorid

Herstellung analog Beispiel 2 aus 10-Bromomethyl-7-chlor-dibenz[b,f]oxepin und N-Methylpropargylamin. Ausbeute: 68%; beige Kristalle; Schmelzpunkt: 189-195°C;
¹H-NMR (CD₃OD, 200MHz): 2.96 (s, 3H); 3.49 (m(t), 1H); 4.15 (m(d), 2H); 4.60 (sbr, 1H); 7.20-7.65 (m, 8H) ; MS: 309 (M⁺, freie Base), 266, 244, 242, 241, 215, 205, 176, 163, 152.

Das Ausgangsmaterial kann beispielsweise auf folgende Weise hergestellt werden:

### a) 2-(3-Chlor-phenoxy)benzaldehyd

Herstellung analog Beispiel 8a) aus 2-Fluor-benzaldehyd und 3-Chlorphenol. Ausbeute: 69%; gelbes Öl nach Kugelrohrdestillation (150-180°C, 0.001 Torr) ; ¹H-NMR (CDCl₃, 200MHz): 6.92-7.38 (m, 6H); 7.55 (m, 1H); 7.95 (m, 1H); 10.48 (s, 1H).

### b) 7-Chlor-dibenz[b,f]oxepin-10-carbonsäure

Herstellung analog Beispiel 9b) aus 2-(3-Chlor-phenoxy)-benzaldehyd. Kristallisiert aus Tertiärbutyl-methylether/Hexan. Ausbeute: 25%; hellgelbe Kristalle; DC (KG; Essigsäureethylester/Hexan = 1:1; UV): R_{f} = 0.15; ¹H-NMR (CDCl₃, 200MHz): 7.05-7.50 (m, 8H); 7.90 (s, 1H).
MS: 274/272 (M⁺).

### c) (7-Chlor-dibenz[b,f]oxepin-10-yl)methanol

Herstellung analog Beispiel 8f) aus 7-Chlor-dibenz[b,f]oxepin-10-carbonsäure. Ausbeute: 94% als Öl; DC (KG; Essigsäureethylester/Hexan = 1:1; UV): R_{f} = 0.38; ¹H-NMR (CDCl₃, 200MHz): 1.72 (sbr, 1H); 4.67 (s, 2H); 6.91 (s, 1H); 7.10-7.40 (m, 7H) ; MS: 260/258 (M⁺), 217/215.

### d) 10-Brommethyl-7-chlor-dibenz[b,f]oxepin

Herstellung analog Beispiel 8g) aus (7-Chlor-dibenz[b,f]oxepin-10-yl)-methanol. Produkt kristallisiert aus Hexan. Ausbeute: 56%; fast weisse Kristalle; Schmelzpunkt: 117-119°C; DC (KG; Essigsäureethylester/Hexan = 1:1; UV): R_{f} = 0.70; ¹H-NMR (CDCl₃, 200MHz): 4.51 (s, 2H); 7.03 (s, 1H); 7.24-7.50 (m, 7H) ; MS: 324/322/320 (M⁺, Br-Cl-Isotopenverteilung), 243/241 (Cl-Isotopenverteilung), 206/205, 178/176.

### Beispiel 11: N-(8-Methoxy-dibenz[b,f]oxepin-10-ylmethyl)-N-methyl-N-prop-2-inyl-aminhydrochlorid

Herstellung analog Beispiel 2 aus 10-Bromomethyl-8-methoxy-dibenz[b,f]oxepin und N-Methyl-propargylamin. Ausbeute: 35%, weisse Kristalle; Schmelzpunkt: Zersetzung >60°C; ¹H-NMR (CDCl₃, 200MHz): 2.33 (t, 1H); 2.42 (s, 3H); 3.48 (d,2H); 3.62 (s, 2H); 3.78 (s, 3H); 6.80-6.90 (m, 2H); 7.05-7.32 (m, 6H).

Das Ausgangsmaterial kann beispielsweise auf folgende Weise hergestellt werden:

### a) 8-Methoxy-dibenz[b,f]oxepin-10-carbonsäure

Herstellung analog Beispiel 9b) aus 2-(4-Methoxy-phenoxy)-benzaldehyd. Kieselgelchromatographie (Laufmittel.: Essigsäureethylester/Hexan = 1:1), danach kristallisiert aus Essigsäureethylester/Hexan = 7:3. Ausbeute: 19%, beige Kristalle; Schmelzpunkt: 150°C; DC (KG; Essigsäureethylester/Hexan = 1:1; UV): R_{f} = 0.58; ¹H-NMR (CDCl₃, 200MHz): 3.80 (s, 3H); 6.92 (dd, 1H); 7.17-7.45 (m, 7H); 8.13 (s, 1H); MS: 268 (M⁺).

### b) (8-Methoxy-dibenz[b,f]oxepin-10-yl)methanol

Herstellung analog Beispiel 8f) aus 8-Methoxy-dibenz[b,f]oxepin-10-carbonsäure. Ausbeute: 96% als braunes Öl; DC (KG; Essigsäureethylester/Hexan = 1:1; UV): R_{f} = 0.31; ¹H-NMR (CDCl₃, 300 MHz): 1.80 (sbr, 1H); 3.78 (s, 3H); 4.69 (s, 2H); 6.82-7.33 (m, 8H) ; MS: 254 (M⁺), 211, 182, 181, 168, 165, 153, 152.

### c) 10-Brommethyl-8-methoxy-dibenz[b,f]oxepin

Herstellung analog Beispiel 8g) aus (8-Methoxy-dibenz[b,f]oxepin-10-yl)-methanol. Produkt kristallisiert aus Hexan/Tertiärbutyl-methylether. Ausbeute: 96%, hellbraune Kristalle; DC (KG; Essigsäureethylester; UV): R_{f} = 0.70; ¹H-NMR (CDCl₃, 200MHz): 3.80 (s, 3H); 4.52 (s, 2H); 6.86-7.37 (m, 8H).

### Beispiel 12: N-(8-Tertiärbutyl-dibenz[b,f]oxepin-10-ylmethyl)-N-methyl-N-prop-2-inyl-aminhydrochlorid

Herstellung analog Beispiel 2 aus 10-Brommethyl-tertiärbutyl-dibenz[b,f]oxepin und N-Methyl-propargylamin. Ausbeute: 24%, beige Kristalle; Schmelzpunkt: 135-145°C; ¹H-NMR (CD₃OD, 200MHz): 1.35 (s, 9H); 2.96 (s, 3H); 3.52 (t, 1H); 4.17 (d, 2H); 7.15-7.60 (m, 9H); El-MS: 331(M⁺), 264, 249, 237, 207.

Das Ausgangsmaterial kann beispielsweise auf folgende Weise hergestellt werden:

### a) 2-(4-Tertiärbutyl-phenoxy)benzaldehyd

Herstellung analog Beispiel 8a) aus 2-Fluor-benzaldehyd und 4-Tertiärbutylphenol. Ausbeute: 77%, gelbes Öl nach HV-Destillation (93-100°C, 0.9 mbar); DC (KG; Hexan/Essigsäureethylester = 1:1; UV): R_{f} = 0.65; ¹H-NMR (CDCl₃, 200MHz): 1.35 (s, 9H); 6.75-7.55 (m, 7H); 7.92 (dd, 1H); 10.50 (s, 1H); MS: 254 (M⁺), 239.

### b) 8-Tertiärbutyl-dibenz[b,f]oxepin-10-carbonsäure

Herstellung analog Beispiel 9b) aus 2-(4-Tertiärbutyl-phenoxy)-benzaldehyd. Kristallisiert aus Hexan Ausbeute: 16%, hellgelbe Kristalle; Schmelzpunkt: 72°C; DC (KG; Hexan/Essigsäureethylester = 4:1; UV): R_{f} = 0.46; ¹H-NMR (CDCl₃, 200MHz): 1.31 (s, 9H); 7.15-7.45 (m, 8H); 7.62 (d, 1H); 8.10 (s, 1H) ; MS: 295 (M⁺), 279, 239.

### c) (8-Tertiärbutyl-dibenz[b,f]oxepin-10-yl)methanol

Herstellung analog Beispiel 8f) aus 8-Methoxy-dibenz[b,f]oxepin-10-carbonsäure. Ausbeute: 30% als Öl nach Chromatographie (Kieselgel; Hexan/Essigsäureethylester = 9:1) und Kugelrohrdestillation (100°C, 0.3 mbar); DC (KG; Hexan/Essigsäureethylester = 9:1; UV): R_{f} = 0.11
¹H-NMR (CDCl₃, 200MHz): 1.30 (s, 9H); 1.72 (sbr, 1H); 4.75 (s, 2H); 6.92 (s, 1H); 7.06-7.45 (m, 7H) ; MS: 280 (M⁺), 265, 237.

### d) 10 -Brommethyl-8-tertiärbutyl-dibenz[b,f]oxepin

Herstellung analog Beispiel 8g) aus (8-Tertiärbutyl-dibenz[b,f]oxepin-10-yl)-methanol. Ausbeute: 85%, braunes Öl; DC (KG; Essigsäureethylester/Hexan = 1:1; UV): R_{f} = 0.58; ¹H-NMR (CDCl₃, 200MHz): 1.31 (s, 9H); 4.58 (s, 2H); 7.05-7.45 (m, 7H); 7.60 (d, 1H); MS: 344/342 (M⁺), 263.

### Beispiel 13: N-(6-Chlor-dibenz[b,f]oxepin-10-ylmethyl)-N-methyl-N-prop-2-inyl-aminhydrochlorid

Herstellung analog Beispiel 2 aus 10-Brommethyl-6-brom-dibenz[b,f]oxepin und N-Methylpropargylamin. Ausbeute: 14%, weisse Kristalle; Schmelzpunkt: 188-190°C; ¹H-NMR (freie Base) (CDCl₃, 200MHz): 2.28 (t, 1H); 2.40 (s, 3H); 3.45 (d, 2H); 3.63 (s, 2H); 6.93-7.50 (m, 8H) ; MS: 310, 312 (M⁺+1, freie Base).

Das Ausgangsmaterial kann beispielsweise auf folgende Weise hergestellt werden:

### a) [2-(2-Chlorphenoxy)phenyl]methanol

Herstellung analog Beispiel 8b) aus 2-(2-Chlor-phenoxy)-benzoesäure im Hochvakuum destilliert. Ausbeute: 53%, gelbes Öl; DC (KG; Essigsäureethylester): R_{f} = 0.74; ¹H-NMR (CDCl₃, 200MHz): 2.60 (sbr, 1H); 4.78 (s, 2H); 6.70-7.50 (m, 8H).
FD-MS: 234, 236 (M⁺).

### b) [2-(2-Chlor-phenoxy)-henyl]-brom-methan

Herstellung analog Beispiel 8c) aus [2-(2-Chlor-phenoxy)-phenyl]-methanol. Kristallisiert aus Hexan Ausbeute: 97% roh, leicht bräunliches Öl; ¹H-NMR (CDCl₃, 200MHz): 4.68 (s, 2H); 6.70 (dd, 1H); 7.02-7.52 (m, 7H); DC (KG; Hexan/Essigsäureethylester = 7:3; UV): R_{f} = 0.69.

### c) [2-(2-Chlor-phenoxy)-phenyl]acetonitril

Herstellung analog Beispiel 8d) aus [2-(2-Chlor-phenoxy)-phenyl]-brommethan. Ausbeute: 99%, roh, braunes Öl; DC (KG; Hexan/Essigsäureethylester = 7:3; UV): R_{f} = 0.52; ¹H-NMR (CDCI3, 200MHz): 3.90 (s, 2H); 6.65-7.53 (m, 8H) ; MS: 295 (M⁺), 279, 239.

### d) 6-Brom-dibenz[b,f]oxepin-10-carbonsäure

Herstellung analog Beispiel 8e) aus [2-(2-Chlor-phenoxy)phenyl]acetonitril. Ausbeute: 85%, gelbe Kristalle; ¹H-NMR (CDCl₃, 200MHz): 6.70-7.50 (m, 7H); 8.32 (dd, 1H).

### e) (6-Brom-dibenz[b,f]oxepin-10-yl)methanol

Herstellung analog Beispiel 8f) aus 6-Brom-dibenz[b,f]oxepin-10-carbonsäure. Ausbeute: 99% roh, braunes Öl; DC (KG; Hexan/Essigsäureethylester = 7:3; UV): R_{f} = 0.07; ¹H-NMR (CDCI3, 200MHz): 3.54 (s, 2H); 6.70-7.49 (m, 8H).

### f) 10-Brommethyl-6-brom-dibenz[b,f]oxepin

Herstellung analog Beispiel 8g) aus (6-Brom-dibenz[b,f]oxepin-10-yl)-methanol. Ausbeute: 8%, braun-oranges Öl; ¹H-NMR (CDCl₃, 200MHz): 4.53 (s, 2H); 7.05-7.60 (m, 8H).

### Beispiel 14: In analoger Weise wie in den Beispielen 1 bis 13 beschrieben kann man auch

N-(1-Fluor-dibenz[b,f]oxepin-10-ylmethyl)-N-methyl-N-prop-2-inyl-amin;
N-Benzyl-N-(dibenz[b,f]oxepin-10-ylmethyl)-amin *alias* 10-Benzylaminomethyldibenz[b,f]oxepin;
N-Benzyl-N-(dibenz[b,f]oxepin-10-ylmethyl)-N-methyl-amin; und
N-(Dibenz[b,f]oxepin-10-ylmethyl)-N-propyl-N-benzyl-amin
und ihre Salze herstellen.

### Beispiel 15: Tabletten, enthaltend je 50 mg N-(Dibenz[b,f]oxepin-10-ylmethyl)-N-methyl-N-prop-2-inyl-amin oder ein Salz, z.B. das Hydrochlorid, davon können wie folgt hergestellt werden:

| Zusammensetzung (10000 Tabletten) | |
|---|---|
| Wirkstoff | 500.0 g |
| Laktose | 500.0 g |
| Kartoffelstärke | 352.0 g |
| Gelatine | 8.0 g |
| Talk | 60.0 g |
| Magnesiumstearat | 10.0 g |
| Siliziumdioxid (hochdispers) | 20.0 g |
| Ethanol | q.s. |

Der Wirkstoff wird mit der Laktose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliziumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 16: Eine sterilfiltrierte wässrige Gelatine-Lösung mit 20 % Cyclodextrinen als Lösungsvermittler, enthaltend je 3 mg N-(Dibenz[b,f]oxepin-10-ylmethyl)-N-methyl-N-prop-2-inyl-amin oder ein Salz, z.B. das Hydrochlorid, davon als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

| | |
|---|---|
| Wirkstoff | 3 mg |
| Gelatine | 150.0 mg |
| Phenol | 4.7 mg |
| dest. Wasser mit 20 % Cyclodextrinen als Lösungsvermittler | 1.0 ml |

Beispiel 17: Zur Herstellung einer sterilen Trockensubstanz zur Injektion, enthaltend je 5 mg N-(Dibenz[b,f]oxepin-10-ylmethyl)-N-methyl-N-prop-2-inyl-amin oder ein Salz, z.B. das Hydrochlorid, davon löst man 5 mg einer der in den vorausgehenden Beispielen genannten Verbindungen der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

Beispiel 18: Für die Herstellung von 10 000 Lacktabletten, enthaltend je 100 mg N-(Dibenz[b,f]oxepin-10-ylmethyl)-N-methyl-N-prop-2-inyl-amin oder ein Salz, z.B. das Hydrochlorid, davon können wie folgt hergestellt werden:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q. s. |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45° während 30 Minuten. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Beispiel 19: In analoger Weise wie in den Beispielen 15 bis 18 beschrieben kann man ferner pharmazeutische Präparate enthaltend eine andere Verbindung gemäss einem der Beispielen 1 bis 14 oder jeweils ein Salz davon herstellen.

## Patentansprüche

1. 10-Aminoaliphatyl-dibenz[b,f]oxepine der Formel I 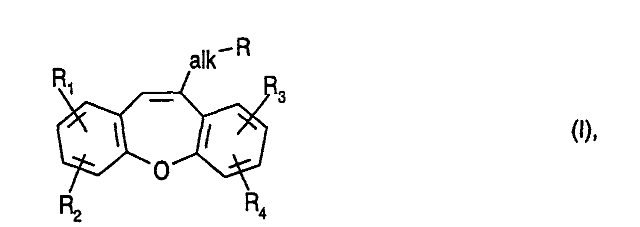 worin
alk Methylen bedeutet,
R Amino, unsubstituiertes oder durch C₁-C₄-Alkyl, C₁-C₄-Alkoxy, Halogen der Atomnummer bis und mit 35 und/oder Trifluormethyl substituiertes Phenyl-C₁-C₄-alkylamino, unsubstituiertes oder durch C₁-C₄-Alkyl, C₁-C₄-Alkoxy, Halogen der Atomnummer bis und mit 35 und/oder Trifluormethyl substituierte N-Phenyl-C₁-C₄-alkyl-N-C₁-C₄-alkyl-amino oder C₂-C₇-Alkenylamino, C₂-C₇-Alkinylamino, N-C₂-C₇-Alkenyl-N-C₁-C₄-Alkyl-amino, N-C₂-C₇-Alkinyl-N-C₁-C₄-alkyl-amino, darstellt und
R₁, R₂, R₃ und R₄ unabhängig voneinander für Wasserstoff, C₁-C₄-Alkyl, C₁-C₄-Alkoxy, Halogen der Atomnummer bis und mit 35 oder Trifluormethyl stehen,
und ihre Salze.

2. Verbindungen gemäss Anspruch 1, worin
alk Methylen ist,
R C₂-C₇-Alkenylamino, C₂-C₇-Alkinylamino, N-C₂-C₇-Alkenyl-N-C₁-C₄-Alkyl-amino oder N-C₂-C₇-Alkinyl-N-C₁-C₄-alkyl-amino darstellt
und R₁ und R₃ unabhängig voneinander Wasserstoff, C₁-C₄-Alkyl, C₁-C₄-Alkoxy, Halogen oder Trifluormethyl und R₂, R₄ Wasserstoff bedeuten,
und ihre Salze.

3. Verbindungen gemäss Anspruch 1, worin
alk Methylen ist,
R C₂-C₇-Alkenylamino, C₂-C₇-Alkinylamino, N-C₂-C₇-Alkenyl-N-C₁-C₄-Alkyl-amino, N-C₂-C₇-Alkinyl-N-C₁-C₄-alkyl-amino oder unsubstituiertes oder durch C₁-C₄-Alkyl, C₁-C₄-Alkoxy, Halogen der Atomnummer bis und mit 35 und/oder Trifluormethyl substituiertes Phenyl-C₁-C₄-alkylamino, wie Benzylamino oder Phenethylamino, darstellt
und R₁, R₂, R₃ und R₄ Wasserstoff bedeuten,
und ihre Salze.

4. N-Allyl-N-(dibenz[b,f]oxepin-10-ylmethyl)-amin;
N-Benzyl-N-(dibenz[b,f]oxepin-10-ylmethyl)-amin;
N-(Dibenz[b,f]oxepin-10-ylmethyl)-N-prop-2-inyl-amin;
N-Allyl-N-(dibenz[b,f]oxepin-10-ylmethyl)-N-methyl-amin;
N-(Dibenz[b,f]oxepin-10-ylmethyl)amin;
N-(1-Chlor-dibenz[b,f]oxepin-10-ylmethyl)-N-methyl-N-prop-2-inyl-amin;
N-Methyl-N-prop-2-inyl-N-(3-trifluormethyl-dibenz[b,f]oxepin-10-ylmethyl)-amin;
N-(7-Chlor-dibenz[b,f]oxepin-10-ylmethyl)-N-methyl-N-prop-2-inyl-amin;
N-(8-Methoxy-dibenz[b,f]oxepin-10-ylmethyl)-N-methyl-N-prop-2-inyl-amin;
N-(8-Tertiärbutyl-dibenz[b,f]oxepin-10-ylmethyl)-N-methyl-N-prop-2-inyl-amin;
N-(6-Chlor-dibenz[b,f]oxepin-10-ylmethyl)-N-methyl-N-prop-2-inyl-amin;
N-(1-Fluor-dibenz[b,f]oxepin-10-ylmethyl)-N-methyl-N-prop-2-inyl-amin
N-Benzyl-N-(dibenz[b,f]oxepin-10-ylmethyl)-N-methyl-amin;
N-(Dibenz[b,f]oxepin-10-ylmethyl)-N-propyl-N-benzyl-amin;
N-(7-Chlor-dibenz[b,f]oxepin-10-ylmethyl)-N-methyl-N-prop-2-inyl-amin
oder jeweils ein Salz davon.

5. N-(Dibenz[b,f]oxepin-10-ylmethyl)-N-methyl-N-prop-2-inyl-amin oder ein Salz davon.

6. Eine Verbindung gemäss einem der Ansprüche 1 bis 5 oder ein pharmazeutisch verwendbares Salz davon zur therapeutischen Behandlung des menschlichen oder tierischen Körpers.

7. Verwendung einer Verbindung gemäss einem der Ansprüche 1 bis 5 oder eines pharmazeutisch verwendbaren Salzes davon, zur Herstellung eines Arzneimittels zur Behandlung von neurodegenerativen Krankheiten.

8. Pharmazeutische Präparate, enthaltend eine Verbindung gemäss einem der Ansprüche 1 bis 5 oder ein pharmazeutisch verwendbares Salz davon neben üblichen pharmazeutischen Hilfs- und Trägerstoffen.

9. Verfahren zur Herstellung von Verbindungen der Formel I 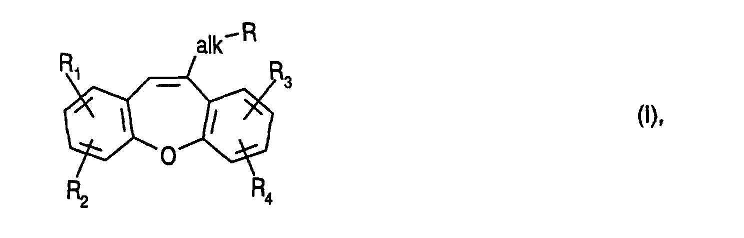 worin alk, R und R₁ bis R₄ wie im Anspruch 1 definiert sind.
und ihrer Salze, dadurch gekennzeichnet, daß man eine Verbindung der Formel II 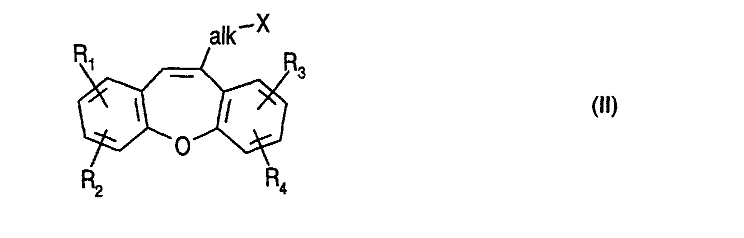 mit einer Verbindung der Formel III
Y-R (III)
kondensiert, worin einer der Reste X und Y reaktionsfähiges verestertes Hydroxy und der andere gegebenenfalls intermediär geschütztes Amino bedeutet und R, R₁, R₂, R₃ und R₄ die angegebenen Bedeutungen haben, und die gegebenenfalls intermediär eingeführte Aminoschutzgruppe wieder abspaltet, und gewünschtenfalls jeweils eine verfahrensgemäss erhältliche Verbindung in eine andere Verbindung der Formel I umwandelt, ein verfahrensgemäss erhältliches Isomerengemische in die Komponenten auftrennt und das gewünschte Isomere isoliert und/oder ein verfahrensgemäss erhältliches Salz in die freie Verbindung oder eine verfahrensgemäss erhältliche freie Verbindung in ein Salz überführt.

## Claims

1. A 10-aminoaliphatyl-dibenz[b,f]oxepine of formula I 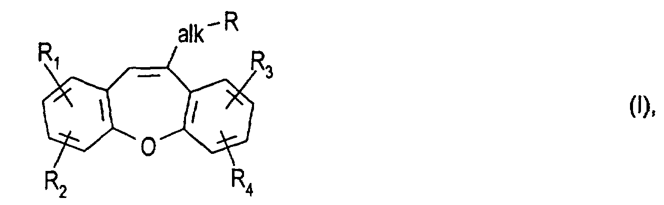 wherein
alk is methylene,
R is amino; phenyl-C₁-C₄alkylamino unsubstituted or substituted by C₁-C₄alkyl, C₁-C₄alkoxy, halogen having an atomic number of up to and including 35 and/or by trifluoromethyl;
N-phenyl-C₁-C₄alkyl-N-C₁-C₄alkylamino unsubstituted or substituted by C₁-C₄alkyl, C₁-C₄alkoxy, halogen having an atomic number of up to and including 35 and/or by trifluoromethyl; or C₂-C₇alkenylamino, C₂-C₇alkynylamino, N-C₂-C₇alkenyl-N-C₁-C₄-alkylamino or N-C₂-C₇alkynyl-N-C₁-C₄alkylamino, and
R₁, R₂, R₃ and R₄ are each, independently of the others, hydrogen, C₁-C₄alkyl, C₁-C₄alkoxy, halogen having an atomic number of up to and including 35 or trifluoromethyl,
or a salt thereof.

2. A compound according to claim 1, wherein
alk is methylene,
R is C₂-C₇alkenylamino, C₂-C₇alkynylamino, N-C₂-C₇alkenyl-N-C₁-C₄alkylamino or N-C₂-C₇-alkynyl-N-C₁-C₄alkylamino, and
R₁ and R₃ are each, independently of the other, hydrogen, C₁-C₄alkyl, C₁-C₄alkoxy, halogen or trifluoromethyl and
R₂ and R₄ are hydrogen,
or a salt thereof.

3. A compound according to claim 1, wherein
alk is methylene,
R is C₂-C₇alkenylamino, C₂-C₇alkynylamino, N-C₂-C₇alkenyl-N-C₁-C₄alkylamino,
N-C₂-C₇alkynyl-N-C₁-C₄alkylamino, or phenyl-C₁-C₄alkylamino, such as benzylamino or phenethylamino, that is unsubstituted or substituted by C₁-C₄alkyl, C₁-C₄alkoxy, halogen having an atomic number of up to and including 35 and/or by trifluoromethyl, and
R₁, R₂, R₃ and R₄ are hydrogen,
or a salt thereof.

4. N-Allyl-N-(dibenz[b,f]oxepin-10-ylmethyl)amine;
N-benzyl-N-(dibenz[b,f]oxepin-10-ylmethyl)amine;
N-(dibenz[b,f]oxepin-10-ylmethyl)-N-prop-2-ynylamine;
N-allyl-N-(dibenz[b,f]oxepin-10-ylmethyl)-N-methylamine;
N-(dibenz[b,f]oxepin-10-ylmethyl)amine;
N-(1-chloro-dibenz[b,f]oxepin-10-ylmethyl)-N-methyl-N-prop-2-ynylamine;
N-methyl-N-prop-2-ynyl-N-(3-trifluoromethyl-dibenz[b,f]oxepin-10-ylmethyl)amine;
N-(7-chloro-dibenz[b,f]oxepin-10-ylmethyl)-N-methyl-N-prop-2-ynylamine;
N-(8-methoxy-dibenz[b,f]oxepin-10-ylmethyl)-N-methyl-N-prop-2-ynylamine;
N-(8-tert-butyl-dibenz[b,f]oxepin-10-ylmethyl)-N-methyl-N-prop-2-ynylamine;
N-(6-chloro-dibenz[b,f]oxepin-10-ylmethyl)-N-methyl-N-prop-2-ynylamine;
N-(1-fluoro-dibenz[b,f]oxepin-10-ylmethyl)-N-methyl-N-prop-2-ynylamine;
N-benzyl-N-(dibenz[b,f]oxepin-10-ylmethyl)-N-methylamine;
N-(dibenz[b,f]oxepin-10-ylmethyl)-N-propyl-N-benzylamine;
N-(7-chloro-dibenz[b,f]oxepin-10-ylmethyl)-N-methyl-N-prop-2-ynylamine;
or in each case a salt thereof.

5. N-(Dibenz[b,f]oxepin-10-ylmethyl)-N-methyl-N-prop-2-ynylamine or a salt thereof.

6. A compound according to any one of claims 1 to 5 or a pharmaceutically acceptable salt thereof for the therapeutic treatment of the human or animal body.

7. The use of a compound according to any one of claims 1 to 5 or of a pharmaceutically acceptable salt thereof in the preparation of a medicament for the treatment of neurodegenerative disease.

8. A pharmaceutical composition comprising a compound according to any one of claims 1 to 5 or a pharmaceutically acceptable salt thereof together with customary pharmaceutical excipients and carriers.

9. A process for the preparation of a compound of formula I 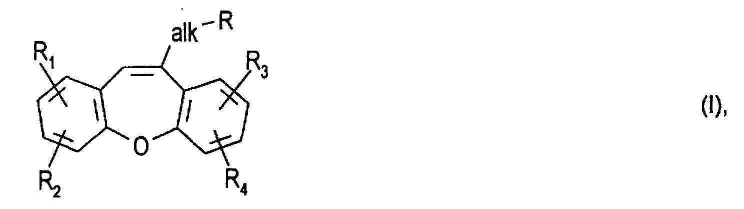 wherein alk, R and R₁ to R₄ are as defined in claim 1, or a salt thereof, which comprises condensing a compound of formula II 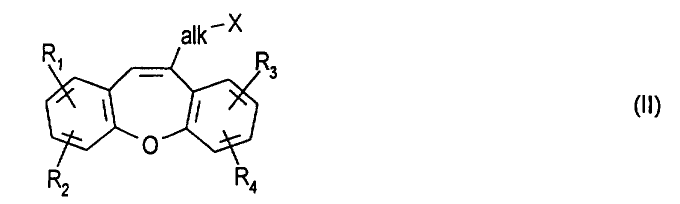 with a compound of formula III
Y-R (III)
wherein one of the radicals X and Y is reactive esterified hydroxy and the other is free or temporarily protected amino, and R, R₁, R₂, R₃ and R₄ are as defined, and removing again the amino-protecting group that may have been temporarily introduced,
and, if desired, in each case converting a compound obtainable in accordance with the process into a different compound of formula I, separating a mixture of isomers obtainable in accordance with the process into its components and isolating the desired isomer, and/or converting a salt obtainable in accordance with the process into the free compound or converting a free compound obtainable in accordance with the process into a salt.

## Revendications

1. La 10-aminoaliphatyl-dibenz[b,f]oxépine de formule I 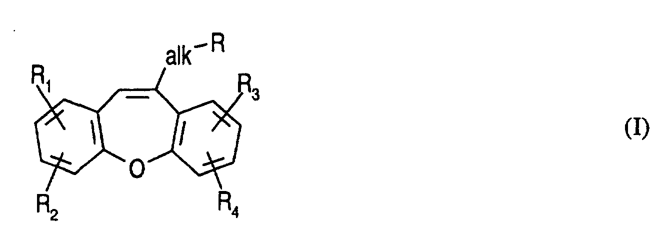 où
alk signifie un groupe méthylène,
R signifie un groupe amino, phényl-C₁-C₄-alkylamino non substitué ou substitué par un groupe C₁-C₄-alkyle, C₁-C₄-alcoxy, un halogène ayant un nombre atomique jusqu'à et incluant 35 et/ou trifluorométhyle, un groupe N-phényl-C₁-C₄-alkyl-N-C₁-C₄-alkylamino non substitué ou substitué par un groupe C₁-C₄-alkyle, C₁-C₄-alcoxy, un halogène ayant un nombre atomique jusqu'à et incluant 35 et/ou trifluorométhyle ou C₂-C₇-alcénylamino, C₂-C₇-alcynylamino, N-C₂-C₇-alcényl-N-C₁-C₄-alkyl-amino, N-C₂-C₇-alcynyl-N-C₁-C₄-alkyl-amino et,
R₁, R₂, R₃ et R₄ indépendamment les uns des autres, signifient l'hydrogène, un groupe C₁-C₄-alkyle, C₁-C₄-alcoxy, un halogène ayant un nombre atomique jusqu'à et incluant 35 ou trifluorométhyle,
et ses sels.

2. Les composés selon la revendication 1, où
alk signifie un groupe méthylène,
R signifie un groupe C₂-C₇-alcénylamino, C₂-C₇-alcynyIamino, N-C₂-C₇-alcényl-N-C₁-C₄-alkyl-amino ou N-C₂-C₇-alcynyl-N-C₁-C₄-alkyl-amino
et R₁ et R₃ indépendamment l'un de l'autre, signifient l'hydrogène, un groupe C₁-C₄-alkyle, C₁-C₄-alcoxy, un halogène ou trifluorométhyle et R₂, R₄ signifient l'hydrogène,
et leurs sels.

3. Les composés selon la revendication 1, où
alk signifie un groupe méthylène,
R signifie un groupe C₂-C₇-alcénylamino, C₂-C₇-alcynylamino, N-C₂-C₇-alcényl-N-C₁-C₄-alkyl-amino, N-C₂-C₇-alcynyl-N-C₁-C₄-alkyl-amino ou phényl-C₁-C₄-alkylamino non substitué ou substitué par un groupe C₁-C₄-alkyle, C₁-C₄-alcoxy, un halogène ayant un nombre atomique jusqu'à et incluant 35 et/ou trifluorométhyle, tel que benzylamino ou phenéthylamino,
et R₁, R₂, R₃ et R₄ signifient l'hydrogène,
et leurs sels.

4. La N-allyl-N-(dibenz[b,f]oxépine-10-ylméthyl)-amine;
la N-benzyl-N-(dibenz[b,f]oxépine-10-ylméthyl)-amine;
la N-(dibenz[b,f]oxépine-10-ylméthyl)-N-prop-2-inyl-amine;
la N-allyl-N-(dibenz[b,f]oxépine-10-ylméthyl)-N-méthyl-amine;
la N-(dibenz[b,f]oxépine-10-ylméthyl)amine;
la N-(1-Chloro-dibenz[b,f]oxépine-10-ylméthyl)-N-méthyl-N-prop-2-inyl-amine;
la N-méthyl-N-prop-2-inyl-N-(3-trifluorométhyl)-dibenz[b,f]oxépine-10-ylméthyl)amine;
la N-(7-chloro-dibenz[b,f]oxépine-10-ylméthyl)-N-méthyl-N-prop-2-inyl-amine;
la N-(8-méthoxy-dibenz[b,f]oxépine-10-ylméthyl)-N-méthyl-N-prop-2-inyl-amine;
la N-(8-tert.-butyl-dibenz[b,f]oxépine-10-ylméthyl)-N-méthyl-N-prop-2-inyl-amine;
la N-(6-chloro-dibenz[b,f]oxépine-10-ylméthyl)-N-méthyl-N-prop-2-inyl-amine;
la N-(1-fluoro-dibenz[b,f]oxépine-10-ylméthyl)-N-méthyl-N-prop-2-inyl-amine;
la N-benzyl-N-(dibenz[b,f]oxépine-10-ylméthyl)-N-méthyl-amine;
la N-(dibenz[b,f]oxépine-10-ylméthyl)-N-propyl-N-benzyl-amine;
la N-(7-chloro-dibenz[b,f]oxépine-10-ylméthyl)-N-méthyl-N-prop-2-inyl-amine
ou un de leurs sels.

5. La N-(dibenz[b,f]oxépine-10-ylméthyl)-N-méthyl-N-prop-2-inylamine ou un de ses sels.

6. Un composé selon l'une quelconque des revendications 1 à 5 ou un de ses sels pharmaceutiquement acceptables pour le traitement thérapeutique du corps humain ou animal.

7. Utilisation d'un composé selon l'une quelconque des revendications 1 à 5, ou d'un de ses sels pharmaceutiquement acceptables, pour la préparation d'un médicament pour le traitement de maladies neurodégénératives.

8. Des compositions pharmaceutiques contenant un composé selon l'une quelconque des revendications 1 à 5 ou un de ses sels pharmaceutiquement acceptables, ensemble avec des excipients et des véhicules pharmaceutiques habituels.

9. Un procédé de préparation des composés de formule I 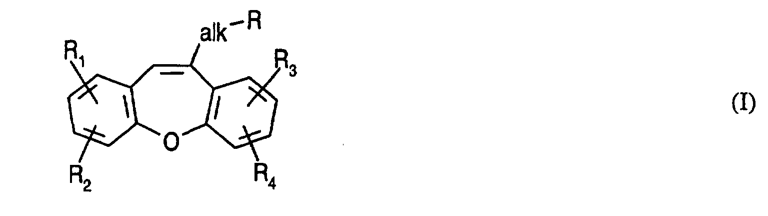 où alk, R et R₁ à R₄ sont tels que définis à la revendication 1
et leurs sels, caractérisé en ce qu'on condense un composé de formule II 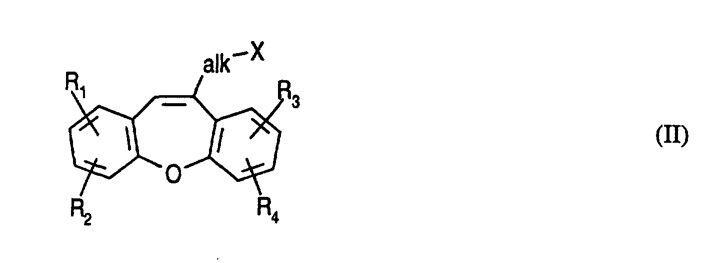 avec un composé de formule III
Y-R (III)
où un des restes X et Y signifie un groupe hydroxy estérifié réactif et l'autre signifie un groupe amino éventuellement temporairement protégé et R, R₁, R₂, R₃ et R₄ ont les significations indiquées et on élimine à nouveau le groupe protecteur du groupe amino éventuellement temporairement introduit, et, si nécessaire, on transforme un composé obtenu selon le procédé en un autre composé de formule I, on sépare un mélange d'isomères obtenu selon le procédé en composants et on isole l'isomère désiré et/ou on transforme un sel obtenu selon le procédé en composé libre ou un composé libre obtenu selon le procédé, en un sel.
